# EUROPEAN PATENT SPECIFICATION

(11) **EP 2 477 638 B1**
(45) Date of publication and mention of the grant of the patent: **25.07.2018**
(21) Application number: 10817876.5
(22) Date of filing: 17.09.2010
(51) Int. Cl.: A61K 35/74, C12N 1/20, A23C 9/152

(54) **PROBIOTIC STABILIZATION**
PROBIOTISCHE STABILISIERUNG
STABILISATION DE PROBIOTIQUE

(30) Priority: 20.09.2009 US 563157
(43) Date of publication of application: 25.07.2012
(73) Proprietor: MJN U.S. Holdings LLC, Chicago, Illinois 60606 (US)
(72) Inventor: RANGAVAJLA, Nagendra, Dublin Ohio 43017 (US); CEVALLOS, Bolivar, Newburgh, IN 47630 (US)
(74) Representative: Gill-Carey, Michael
(86) International application number: PCT/US2010/049225
(87) International publication number: WO 2011/035093

(56) References cited:
- WO-A1-2006/122965
- WO-A1-2006/130204
- WO-A1-2008/076975
- US-A1- 2006 286 252
- US-A1- 2007 116 826
- US-A1- 2007 116 826
- US-A1- 2008 219 961
- US-A1- 2008 220 470
- US-A1- 2008 220 470
- US-A1- 2009 092 590
- US-A1- 2009 203 592
- US-A1- 2009 203 592
- US-A1- 2009 215 718
- US-A1- 2009 215 718

## Description

### TECHNICAL FIELD

The present disclosure relates to the stabilization of biological material for ingestion by an individual. More particularly, the present disclosure relates to a stabilized bacterial mixture comprising hydrolyzed mammal, especially bovine, protein so that a probiotic organism may have improved stability during product distribution and storage.

### BACKGROUND ART

There are currently a variety of compositions for supplementing the gastrointestinal tracts of both humans and animals. These supplements may be provided to alter, reduce or increase the microflora within the individual's gut so as to cause a desired effect on digestion. An example of lessening allergies in infants is disclosed in WO 2006/130204, issued to Rangavajla et al. Herein, the use of low-lactose partially hydrolyzed infant formula is disclosed. Further examples on the use of hydrolyzed proteins in dietary compositions providing various health benefits are provided by Beermann et al. (US 2009/0203592) and Edens et al. (US 2008/0220470). In addition to the above examples concerning hydrolyzed proteins in dietary compositions, US 2009/0215718 by van Laere et al. discloses a manner to obtain a sustained and lower glucose release after consumption. Ideally, supplementation may cultivate an improved microflora for individuals, including humans, based upon the alteration of specific bacteria within the human's gastrointestinal (GI) tract. This style of supplementation may be conducted through the use of probiotics which are understood to be live microorganisms that when administered in effective amounts, confer a health or nutritional benefit to the host. One of the more common types of probiotics is a lactic acid bacterium which is able to convert sugars and other carbohydrates into lactic acid. This conversion lowers the pH within the gut of the host and provides fewer opportunities for harmful organisms to grow and cause problems through gastrointestinal infections.

A common technological challenge is introducing the probiotics into the host in an appropriate manner both for the maintenance of the probiotics as well as for the health and enjoyment of an individual. A background reference is US 2007/116826. Disclosed are functional sweetener compositions comprising non-caloric or low-caloric natural and/or synthetic high-potency sweeteners and methods for making and using them. Specifically, US 2007/116826 teaches compositions comprising a probiotic or prebiotic, a sweetener, and a sweet taste improving composition Current technologies concerning maintenance of probiotics include the utilization of encapsulation and stabilization techniques for shielding the probiotics with a protective layer or matrix so that the protected microbe may pass to the appropriate location within the individual's GI tract. For example, in Batich et al. (U.S. Patent No. 5,286,495), a process for microencapsulating cells is provided so that oxalate-degrading enzymes and bacteria may be encapsulated for both enteric and intraperitoneal administration. According to Batich et al., bacteria and enzymes can be successfully encapsulated in either alginate microcapsules or cellulose acetate phthalate microspheres. The model suggests that viability remains for the bacteria and enzymes so that the encapsulated cells reach the appropriate gastric region of the animal.

In U.S. Patent Application No. 5,733,568 issued to Ford, micro-encapsulated *Lactobacilli* bacteria are administered to the skin to treat or prevent recurrent skin infections. *Lactobacillus* species are mixed with a glucose saline solution and gently stirred with a sodium alginate solution prior to being forced through a needle and dried to create gelled droplets. Other methods of encapsulation may include the addition of bacteria to a suspension of polyvinyl povidone or hydroxypropyl methylcellulose for encapsulating the bacteria.

In U.S. Patent No. 6,465,706, issued to Rogers et al., encapsulation of microbes is described for use in biodecontamination. Rogers et al. asserts that suitable encapsulation materials include natural or synthetic polymeric binders that encompasses both gels and foams as well as gelatin polymers.

Although there have been developments concerning encapsulation and stabilization techniques for containing microorganisms for delivery into the digestive system of animals, there has been little development in encapsulation or stabilization techniques that protect the viability of probiotics during distribution and storage. There is a need for a stabilization technique where circumstances preclude refrigeration, and further where such formulations may be exposed to various environments, especially those associated with tropical climates. In addition, the inherent moisture of the product poses a challenge in that probiotics generally are sensitive to water, especially in combination with high temperature. To date, no technology or technique has been identified to deliver a significant protection to probiotics under intermediate moisture conditions (i.e. water activity of about 0.2 and higher, and up to about 0.4 or higher) and high temperatures during distribution and storage (i.e. temperatures of at least about 30°C, and up to and above 40°C) when incorporated into nutritional agents.

In particular, probiotics can provide a variety of benefits to the host, such as maintaining a healthy gastrointestinal flora, enhancing immunity, protecting against diarrhea, atopic dermatitis and other diseases, etc. As such, there is a need for probiotics to be administered in various geographic locations, including tropical climates, where the viability of the probiotic could be compromised. Conventional encapsulation and stabilization techniques are generally considered suitable only for non-humans and possess a chemical makeup which is ill-suited for infant formulas and use by children, or have poor stability characteristics that significantly limit commercial opportunities.

What is desired therefore, is a stabilization technique and a stabilized bacterial mixture using acceptable ingredients for either an infant formula or children's nutrition, the stabilized mixture allowing for improved stability properties so that probiotics may be distributed in a wide variety of geographical locations and climates while maintaining a useful shelf-life. Further desired is a stabilization technology for the protection of *Lactobacillus rhamnosus* for use in infant formula, supplements and children's products. Indeed, a combination of characteristics, including improved stability combined with nutritional factors, provide an improved stabilization mixture applicable for prenatal, infant, and children's nutrition.

### DISCLOSURE OF THE INVENTION

The present disclosure provides a stabilized mixture that may be used for improving the stability of a biological material. In embodiments of the disclosure, the stabilized substrate may be a probiotic where the various health benefits associated with the stabilized probiotic may be conferred to the individual upon ingesting a composition containing the stabilized microbe.

While probiotics have been recognized as nutritionally beneficial, it is generally accepted that most probiotics provide an optimal beneficial effect only when the microorganism is alive. For optimal effectiveness, the probiotics need to survive the conditions of manufacturing into a consumable item such as a food or beverage as well as the subsequent shipping and storage time prior to the product being ingested for the introduction of the probiotic into the GI tract. Most conventional probiotic compositions utilize an extremely high count of bacterial cells, with the understanding that a significant number of cells ultimately lose viability and die during the manufacturing process, transport, and storage. Current encapsulation and stabilization techniques provide some protection for the probiotics yet do not provide a desired stability while simultaneously being functional for infant formulas and children's products.

By practice of the present disclosure, hydrolyzed mammalian protein is incorporated into a stabilized mixture, which strengthens the matrix and increases the stability of the probiotics. As a result, infant- and children-compatible probiotics can be stabilized with a mixture including hydrolyzed mammalian protein and used in multiple environments as the probiotics exhibit an improved viability. Advantageously, probiotics stabilized by the present disclosure can be incorporated into nutritional products and shipped over extended distances, as the probiotics will maintain viability even after extended transportation and storage time due to the improved stability of the stabilized matrix.

More in detail, the invention relates to an ingestible composition comprising a stabilized probiotic contained in a mixture of hydrolyzed mammalian protein, as defined in claim 1.

While the stabilized matrix may be utilized for a variety of substances, in a preferred embodiment, it is utilized for probiotics such as *Lactobacillus rhamnosus. Lactobacillus rhamnosus* is understood to possess relatively good bio-stability while having a high avidity for human intestinal mucosal cells. In use as a probiotic, *Lactobacillus rhamnosus* is thought to colonize the digestive tract and balance intestinal microflora.

In creating the stabilized mixture for the probiotic, a hydrolyzed mammalian protein may be used to increase and strengthen the matrix around the probiotic. The hydrolyzed mammalian protein may include extensively hydrolyzed casein as well as other hydrolyzed mammalian proteins and is hypothesized to provide the increase in strength due to the characteristics associated with the short chain peptides comprising the hydrolyzed protein. The hydrolyzed protein may be achieved by boiling mammalian protein in a strong acid or strong base or through an enzymatic degradation technique so as to break the protein down into shorter sequences of its component amino acids.

Other components of the stabilized bacterial mixture may include carbohydrates encompassing monosaccharides, disaccharides and polysaccharides. Trehalose, a disaccharide, may be utilized as a carbohydrate in the stabilization mixture. It is generally known to have a relatively high water retention capacity and can form a gel phase. Trehalose is used by plants and animals during prolonged periods of desiccation and is understood to be an effective agent in protecting biological materials in arid environments. Inulin, a plant-created polysaccharide, is indigestible by the human enzymes ptyalin and amylase, as they are adapted for the digestion of starch. As a result, inulin is a useful stabilization mixture ingredient as it passes through much of the digestive system intact and is metabolized by the microflora in the colon, thus stimulating the growth of the gut bacterial flora. In addition, inulin is used to increase the glass transition temperature of the matrix, upward shifting the temperature at which the matrix turns rubbery; therefore preventing an increase in water mobility and probiotic degradation at lower temperatures.

A compound binder may also be used in the stabilization mixture, an example being the chemical compound sodium alginate. Sodium alginate can form a gum-like material and increase the viscosity of mixtures to which it is added. Additionally, the compound may also provide for greater ease in mixing of the components together as sodium alginate may also possess emulsifier characteristics.

The stabilization mixture provides for improved stability of the probiotic, meaning that a greater percentage of the probiotic cells are viable after processing, transportation and storage conditions. Specifically, the shelf life of probiotics is improved when compared to encapsulation and stabilization techniques using intact protein, hydrolyzed non-mammalian protein or protein which is not as extensively hydrolyzed as used herein.

The stabilization mixture of the present disclosure may be used in a multiplicity of processes in forming a stabilized probiotic product. These processes include freezing, freeze-drying, ambient air drying, vacuum drying, spray drying, low temperature drying, high temperature drying and a combination thereof. The resulting stabilized probiotic, whether alone or integrated into a nutritional product, possesses effective viability in a wide range of temperatures and conditions while displaying improved shelf-life. Furthermore, the stabilized probiotic may be incorporated into a variety of prenatal, infant and children's nutritional products for improving their gut microflora while simultaneously providing nutrition to the infant or child.

Accordingly, in one embodiment, the disclosure is a stabilization of biological material providing for increased stability of the biological material.

Still another embodiment is a stabilization mixture for a probiotic.

Another embodiment is a stabilization mixture for a probiotic comprising hydrolyzed mammalian protein.

Yet another embodiment is a mixture for stabilizing a probiotic comprising one or more carbohydrates, a compound binder and hydrolyzed mammalian protein.

A further embodiment is a method of increasing the shelf life of probiotics comprising stabilizing the probiotic with a stabilization mixture including hydrolyzed mammalian protein.

These aspects and others that will become apparent to the skilled artisan upon review of the following description can be accomplished by providing a mixture including hydrolyzed mammalian protein for the stabilization of biological material, such as probiotics, to provide for increased stability of the biological material, resulting in the improved, long-term viability of the biological material. In an embodiment, the stabilization mixture advantageously provides for an extension of the shelf-life of probiotics such as *Lactobacillus rhamnosus* when compared to the use of non-hydrolyzed or hydrolyzed non-mammalian protein. The stabilization mixture may be combined with the probiotic in a variety of methods including freeze drying, air drying, vacuum drying, spray drying and combinations thereof for preserving the probiotic.

It is to be understood that both the foregoing general description and the following detailed description provide embodiments of the disclosure and are intended to provide an overview or framework of understanding to the nature and character of the disclosure as it is claimed.

### BEST MODE FOR CARRYING OUT THE INVENTION

The present disclosure provides a novel stabilization formula that provides stability and protection to biological materials for improving the viability of the target microorganism. The present disclosure includes a stabilization mixture comprising a hydrolyzed mammalian protein, which in certain embodiments is combined with one or more carbohydrates and a compound binder which together provide a protective matrix resulting in an increased shelf-life over other encapsulation and stabilization products utilizing non-hydrolyzed or hydrolyzed non-mammalian proteins.

### DEFINITIONS

The term "probiotic" means a microorganism with low or no pathogenicity that exerts beneficial effects on the health of the host.

As used herein, the term "infant" is generally defined as a human from about birth to 12 months of age.

A "pre-term infant" is an infant born after less than about 37 weeks gestation.

A "full-term infant" as used herein means an infant born after at least about 37 weeks gestation.

"Children" are defined as humans over the age of about 12 months to about 12 years old.

An "effective amount" as used herein is generally defined as an amount of an agent that provides an observable result within the subject administered thereto.

The term "nutritional product" as used herein encompasses any consumable matter of either plant, animal or synthetic sources that may contain nutrients.

### PREFERRED EMBODIMENTS

In the practice of the present disclosure, hydrolyzed mammalian protein is utilized as a component within a stabilization mixture for stabilizing biological material. As used herein, hydrolyzed protein is used to describe a degraded or decomposed protein molecule so that multiple peptide fragments are provided. Within the present disclosure, hydrolyzed protein and protein hydrolysate are used interchangeably to describe products of protein hydrolysis; extensively hydrolyzed protein is used to describe products of protein hydrolysis where at least 20%, more preferably at least 70%, and most preferably at least about 90%, of the hydrolyzed protein has a molecular weight of less than 2000 Daltons.

Hydrolyzed mammalian protein can be created from a variety of mammalian protein sources, including milk products and animal products. It may be created through a process of acid hydrolysis where mammalian protein is subjected to a strong acid and heated until the desired size ranges of amino acid fragments are created. Further types of protein hydrolysis include the use of enzymatic agents which digest protein molecules in creating shorter chains of amino acids. Common processes for hydrolyzing protein are known in the art and described in U.S. Patent No. 4,377,602 issued to Conrad; U.S. Patent No. 4,443,540 issued to Chervan et al.; U.S. Patent No. 4,545,933 issued to Ernster; U.S. Patent No. 4,757,007 issued to Satoh et al.; U.S. Patent No. 4,873,108 issued to De Rooij et al.; U.S. Patent No. 5,401,527 issued to Brown et al.; U.S. Patent No. 6,214,585 issued to Kwong et al.; and U.S. Patent No. 6,221,423 issued to Cho et al.

Mammalian proteins that may be hydrolyzed for use in the stabilization mixture of the present disclosure include egg proteins, animal proteins, poultry, meat, serum albumen, glycol proteins, collagen, gelatin, milk proteins, casein, whey protein, albumen and others. In a preferred embodiment, the mammalian protein is a bovine protein. As previously defined, the hydrolyzed mammalian protein of any of the above types is, in a preferred embodiment, extensively hydrolyzed, meaning at least about 70% of the hydrolyzed protein yielding peptides having a molecular weight of less than about 2,000 Daltons.

In one embodiment, the hydrolyzed mammalian protein comprises hydrolyzed casein having over about 80%, advantageously over about 90%, of the peptides with a molecular weight of less than about 2,000 Daltons. Casein is understood to be a phospho-protein which comprises almost 80% of the total protein in bovine milk. The protein includes no disulfide bridges and as a result has little secondary or tertiary structure. Non-hydrolyzed casein includes casein variants having a molecular weight in the range of from about 19,000 Daltons to about 68,000 Daltons. One embodiment of a mammalian protein hydrolysate, such as casein hydrolysate, which may be used in practice of the present disclosure is one in which over 90% of the peptides have a molecular weight of less than 1,000 Daltons, with over 97% having a molecular weight of less than 2,000 Daltons. Less than 0.3% of the mammalian protein hydrolysate in certain embodiments is over 5,000 Daltons, illustrating that virtually all of the protein was hydrolyzed.

The use of the hydrolyzed mammalian proteins including the aforementioned hydrolyzed casein provides superior protection to probiotics, including *Lactobacillus rhamnosus,* beyond the protection observed through the use of larger peptide fragments or whole proteins. While not being bound by any theory, one possibility for the increased protection to the probiotics against both moisture and heat may involve the increase in the zeta potential of the surface resulting from the hydrolyzed mammalian protein. The zeta potential is a value which indicates the degree of repulsion between adjacent similarly charged particles within dispersion. Smaller compounds and molecules possess a high zeta potential which confers stability as the solution or dispersion will resist aggregation. Conversely, when the zeta potential is low attraction exceeds repulsion and the dispersion may break and flocculate. While hydrophobicity decreases, the magnitude of the zeta potential increases with an increasing degree of hydrolysis. The high degree of hydrolysis providing for many short peptide sequences may increase the zeta potential of the protein interface in contact with the probiotic and thus increase the stability of the biological agent to both heat and humidity. Of course, this does not explain the surprising differences between hydrolyzed mammalian and non-mammalian proteins.

In certain embodiments, the majority component of the stabilization mixture based on a dry weight basis is one or more carbohydrates, which may include polysaccharides, disaccharides and monosaccharides. Examples of monosaccharides may include glucose, fructose, ribose, galactose; disaccharides may include trehalose, sucrose, maltose, lactose, cellobiose; and polysaccharides may include glucose, fructose and galactose polymers, amylose, glycogen, cellulose, inulin, raffinose and stachyose, among other examples. In some embodiments, the carbohydrates may include disaccharides such as sucrose and trehalose, which are known to assist plant and microbial life during periods of low water availability. Trehalose, also known as mycose, is synthesized by fungi, plants and certain animals and functions as a key carbohydrate in anhydrobiosis where biological organisms withstand prolonged periods of desiccation. Physically, trehalose can form a gel phase and is useful in forming the protective matrix around the probiotic. A further embodiment provides that inulin may be included and provides a soluble fiber often considered as a prebiotic in nutritional applications. Advantageously, inulin passes through the stomach and duodenum undigested where it is available for digestion by the gut bacteria flora. The incorporation of inulin may help protect the probiotic from digestive enzymes and high acidity until the probiotic is properly located with the bacteria flora of the gut where inulin may be digested.

A further component of the stabilization mixture in some embodiments can be a compound binder (also termed a gelling agent) which may act as a thickener and produce a gel-like consistency. Examples of compound binders include alginates such as sodium alginate, chitosan, carboxymethylcellulose, among others. The incorporation of the compound binder provides for the formation of a viscous consistency providing for efficient matrix formation and a structural quality suitable for subsequent drying.

The stabilization mixture may also include additional ingredients that provide further benefits to either the probiotic or the individual ingesting the stabilized probiotic. These ingredients may comprise minerals, vitamins, antioxidants, trace elements, sterols, antioxidants, fatty acids, functional molecules, and combinations thereof. Other ingredients may include pectin, resistant starches, high amylose starches, guar, and locust bean gum, agar, xanthan, carrageenans, glucans, fructo-oligosaccharides, polyfructoses, maltodextrins, and combinations thereof.

Foaming of the mixture can be controlled by decreasing the amount of air present in the slurry. In certain embodiments, this can be achieved by mixing under vacuum so as to yield target slurry densities in the range of 1.0 to 1.2 g/cm³.

The stabilization mixture may be used to provide stability to a probiotic organism which may exert a beneficial effect on the health and welfare of individuals. Examples of suitable probiotics include but are not limited to yeasts such as *Saccharomyces cereviseae,* molds such as *Aspergillus, Rhizopus, Mucor,* and bacteria such as *Lactobacillus.* Specific examples of suitable probiotic micro-organisms are: *Aspergillus niger, A. oryzae, Bacillus coagulans, B. lentus, B. licheniformis, B. mesentericus, B. pumilus, B. subtilis, B. natto, Bifidobacterium adolescentis, B. animalis, B. breve, B. bifidum, B. infantis, B. lactis, B. longum, B. pseudolongum, B. thermophilum, Candida pintolepesii, Clostridium butyricum, Enterococcus cremoris, E. diacetylactis, E. faecium, E. intermedius, E. lactis, E. muntdi, E. thermophilus, Lactobacillus acidophilus, L. alimentarius, L. amylovorus, L. crispatus, L. brevis, L. case, L. curvatus, L. cellobiosus, L. delbrueckii ss. bulgaricus, L. farciminis, L. fermentum, L. gasseri, L. helveticus, L. lactis, L. plantarum, L. johnsonii, L. reuteri, L. rhamnosus, L. sakei,* and *L. salivarius.*

In an embodiment of the present disclosure *Lactobacillus rhamnosus* GG is utilized as a probiotic which may be stabilized by the present disclosure. *Lactobacillus rhamnosus* GG is described in U.S. Patent Application 4,839,281, issued to Sherwood et al. Notably, Sherwood et al. describes *Lactobacillus rhamnosus* GG as being a species in which the bacteria have avid adherence to intestinal cells while being simultaneously able to survive at low pHs and produce large amounts of lactic acid.

The selected probiotic is preferably concentrated to a wet paste-like consistency prior to combining with the stabilization mixture of the present disclosure. Starting with probiotics in dry form is also an alternative. Concentration levels of selected probiotics include concentrations of from about 3X to about 20X though may include lesser or greater concentrations depending upon the specific probiotic biomass and subsequent processing steps. Generally, the preparation of a stabilized probiotic includes the steps of concentrating the selected probiotic or probiotics; providing components of the stabilization mixture in desired quantities; mixing the stabilization mixture with the concentrated probiotic; drying the stabilized probiotic and either packaging or combining the stabilized probiotic into a nutritional product which may include an infant formula.

In order to further illustrate the principles and operations of the present disclosure, the following example is provided. However, this example should not be taken as limiting in any regard.

### EXAMPLE

*Lactobacillus rhamnosus* GG (LGG) is grown in a fermenter. The biomass is subsequently washed with buffer and centrifuged to obtain a LGG moist pellet. A stabilization mixture is pre-blended comprising on a dry weight basis approximately 75% trehalose, about 16.7% hydrolyzed casein, 5.2% inulin and approximately 3.1% sodium alginate. At a temperature of 37°C, the LGG moist pellet is mixed with the stabilization mixture and enough water to yield a total solids content of approximately 55%. The slurry is mixed under vacuum to yield a density of around 1.1 g/cm³.

The combination of stabilization mixture and LGG is then either vacuum-dried or freeze-dried to a final moisture content of approximately 3%. It is preferred for the mixture to be spread in a tray at a load ranging from 1076 g/m² to 1614 g/m² (100g/ft² to 150g/ft²). The mixture is dried ensuring product temperature is below 30°C during removal of 80% of the total water. Temperature during removal of the remaining 17% moisture should not exceed 50°C. The dried, stabilized LGG may subsequently be ground and size selected through the use of sieves to obtain a product having a desirable size.

In optimizing the stabilization for probiotic, the multiple constituents may be varied in some embodiments. Carbohydrates may comprise from about 70% to about 85% of the stabilization mixture on a dry basis; the hydrolyzed mammalian protein may comprise from about 10% to about 20% of the stabilization mixture on a dry basis and the compound binder may comprise from about 0% to about 10% (i.e., up to about 10%) of the stabilization mixture on a dry basis.

The stabilized probiotic, with over 70% of the matrix protein having a molecular weight of less than 2,000 Daltons, may be packaged and sold commercially or may be instead combined with a variety of nutritional products. Such nutritional products may include both infant formulas and children's products useful for applications where one desires to incorporate a probiotic into a nutritional product that necessitates an improved shelf-life and stability.

One type of nutritional product for combination with a stabilized probiotic may be an infant formula. The term "infant formula" applies to a composition in liquid or powdered form that satisfies the nutrient requirements of an infant by being a substitute for human milk. In the United States, the content of an infant formula is dictated by the federal regulations set forth at 21 C.F.R. §§100, 106 and 107. These regulations define macronutrient, vitamin, mineral, and other ingredient levels in an effort to simulate the nutritional and other properties of human breast milk. Another nutritional product may be a human milk fortifier, meaning it is a composition which is added to human milk in order to enhance the nutritional value of human milk. As a human milk fortifier, the composition may be in powder or liquid form in combination with the stabilized probiotic.

The stabilized probiotic of the disclosure may be combined with a nutritional product that provides minimal, partial, or total nutritional support. The compositions may be nutritional supplements or meal replacements. The compositions may be administered in conjunction with a food or nutritional composition. The compositions containing the stabilized probiotic can either be intermixed with the food or other nutritional compositions prior to ingestion by the subject or can be administered to the subject either before or after ingestion of a food or nutritional composition. The compositions may be administered to preterm infants receiving infant formula, breast milk, a human milk fortifier, or combinations thereof.

The compositions for combination with the stabilized probiotic may, but need not, be nutritionally complete. The skilled artisan will recognize "nutritionally complete" to vary depending on a number of factors including, but not limited to, age, clinical condition, and dietary intake of the subject to whom the term is being applied. In general, "nutritionally complete" means that a nutritional composition provides adequate amounts of all carbohydrates, lipids, essential fatty acids, proteins, essential amino acids, conditionally essential amino acids, vitamins, minerals, and energy required for normal growth. As applied to nutrients, the term "essential" refers to any nutrient which cannot be synthesized by the body in amounts sufficient for normal growth and to maintain health and which therefore must be supplied by the diet. The term "conditionally essential" as applied to nutrients means that the nutrient must be supplied by the diet under conditions when adequate amounts of the precursor compound is unavailable to the body for endogenous synthesis to occur.

The composition which is "nutritionally complete" for the preterm infant will, by definition, provide qualitatively and quantitatively adequate amounts of all carbohydrates, lipids, essential fatty acids, proteins, essential amino acids, conditionally essential amino acids, vitamins, minerals, and energy required for growth of the preterm infant. The composition which is "nutritionally complete" for the term infant will, by definition, provide qualitatively and quantitatively adequate amounts of all carbohydrates, lipids, essential fatty acids, proteins, essential amino acids, conditionally essential amino acids, vitamins, minerals, and energy required for growth of the term infant. The composition which is "nutritionally complete" for a child will, by definition, provide qualitatively and quantitatively adequate amounts of all carbohydrates, lipids, essential fatty acids, proteins, essential amino acids, conditionally essential amino acids, vitamins, minerals, and energy required for growth of a child.

The stabilized probiotic created by the present disclosure may be combined with a nutritional composition provided in any form known in the art, including a powder, a gel, a suspension, a paste, a solid, a liquid, a liquid concentrate, or a ready-to-use product. In one combination, the nutritional composition is an infant formula, especially an infant formula adapted for use as sole source nutrition for an infant.

The nutritional products described for combining with the stabilized probiotic may be administered enterally. As used herein, "enteral" means through or within the gastrointestinal, or digestive, tract, and "enteral administration" includes oral feeding, intragastric feeding, transpyloric administration, or any other introduction into the digestive tract.

Suitable fat or lipid sources may be any known or used in the art, including but not limited to, animal sources, e.g., milk fat, butter, butter fat, egg yolk lipid; marine sources, such as fish oils, marine oils, single cell oils; vegetable and plant oils, such as corn oil, canola oil, sunflower oil, soybean oil, palmolein, coconut oil, high oleic sunflower oil, evening primrose oil, rapeseed oil, olive oil, flaxseed (linseed) oil, cottonseed oil, high oleic safflower oil, palm stearin, palm kernel oil, wheat germ oil; medium chain triglyceride oils and emulsions and esters of fatty acids; and any combinations thereof.

Bovine milk protein of the nutritional product may include, but are not limited to, milk protein powders, milk protein concentrates, milk protein isolates, nonfat milk solids, nonfat milk, nonfat dry milk, whey protein, whey protein isolates, whey protein concentrates, sweet whey, acid whey, casein, acid casein, caseinate (e.g. sodium caseinate, sodium calcium caseinate, calcium caseinate) and any combinations thereof.

The stabilized probiotic may be combined with a nutritional product having its proteins provided as intact proteins. In other embodiments, the proteins of the nutritional product are provided as a combination of both intact proteins and partially hydrolyzed proteins, with a degree of hydrolysis of between about 4% and 10%. Certain of these embodiments can be extremely hypoallergenic as both the stabilizer and the protein of the nutritional product contain only hydrolyzed protein. In yet another embodiment, the nutritional product may be supplemented with glutamine-containing peptides.

The whey:casein ratio of the protein source of the nutritional product may be similar to that found in human breast milk. In an embodiment, the protein source of the nutritional product comprises from about 40% to about 80% whey protein. In another embodiment, the protein source may comprise from about 20% to about 60% caseins.

When the stabilized probiotic is combined with the nutritional product, the composition may include an amount of probiotics from about 10⁴ to about 10¹⁰ colony forming units (cfu) per kg body weight per day. In other embodiments, the amount of the probiotic within the stabilized probiotic may vary from about 10⁶ to about 10⁹ cfu per kg body weight per day. In even further embodiments the composition of the nutritional product and stabilized probiotic may include an amount of probiotics from about of at least about 10⁶ cfu per kg body weight per day. In another embodiment, the concentration of the probiotic, for instance LGG, in the micromatrix is from about 10⁶ to about 10¹⁴ cfu per g, more preferably from about 10⁹ to about 10¹¹ cfu per g.

In an embodiment, the stabilized probiotic(s) may be viable or non-viable, while viable are preferred. As used herein, the term "viable", refers to live microorganisms, that is, comprising viable microbial cells. The term "non-viable" or "non-viable probiotic" means non-living probiotic microorganisms, their cellular components and metabolites thereof. Such non-viable probiotics may have been heat-killed or otherwise inactivated but may retain some ability to favorably influence the health of the host. The stabilized probiotics useful in the present disclosure may be naturally-occurring, synthetic or developed through the genetic manipulation of organisms, whether such new source is now known or later developed. Additionally, the nutritional product may include non-stabilized probiotics with the final composition including some stabilized probiotics and some non-stabilized probiotics.

The stabilized probiotics may also be combined with a nutritional composition containing one or more prebiotics. The term "prebiotic" as used herein refers to indigestible food ingredients which exert health benefits upon the host. Such health benefits may include, but are not limited to, selective stimulation of the growth and/or activity of one or a limited number of beneficial gut bacteria, stimulation of the growth and/or activity of ingested probiotic (stabilized or not) microorganisms, selective reduction in gut pathogens, and favorable influence on gut short chain fatty acid profile. Such prebiotics may be naturally-occurring, synthetic, or developed through the genetic manipulation of organisms and/or plants, whether such new source is now known or developed later. Prebiotics may include oligosaccharides, polysaccharides, and other prebiotics that contain fructose, xylose, soya, galactose, glucose and mannose. More specifically, prebiotics useful in the present disclosure may include lactulose, lactosucrose, raffinose, gluco-oligosaccharide, inulin, polydextrose, polydextrose powder, galacto-oligosaccharide, fructo-oligosaccharide, isomalto-oligosaccharide, soybean oligosaccharides, lactosucrose, xylo-oligosaccharide, chito-oligosaccharide, manno-oligosaccharide, aribino-oligosaccharide, siallyl-oligosaccharide, fuco-oligosaccharide, and gentio-oligosaccharides, and combinations thereof.

In some compositions of nutritional products including stabilized probiotics, the total amount of prebiotics present in the nutritional composition may be from about 1.0 g/L to about 10.0 g/L of the composition (in the liquid form). As noted, the total amount of prebiotics present in the nutritional composition may be from about 2.0 g/L and about 8.0 g/L of the composition. At least 20% of the prebiotics should, in a preferred embodiment, comprise galacto-oligosaccharide (GOS).

In addition to galacto-oligosaccharide, the prebiotic composition can also comprise polydextrose (PDX). If polydextrose is used as a prebiotic, the amount of polydextrose in the nutritional composition may, in an embodiment, be within the range of from about 1.0 g/L to about 4.0 g/L.

The amount of galacto-oligosaccharide in the nutritional composition may be from about 0.2 mg/100 Kcal to about 1.0 mg/100 Kcal. In other compositions, the amount of galacto-oligosaccharide in the nutritional composition may be from about 0.1 mg/100 Kcal to about 0.5 mg/100 Kcal. If polydextrose is used as a prebiotic, the amount of polydextrose in the nutritional composition may, in an embodiment of the composition including stabilized probiotics, be within the range of from about 0.1 mg/100 Kcal to about 0.5 mg/100 Kcal. In another composition, the amount of polydextrose may be about 0.3 mg/100 Kcal. Galacto-oligosaccharide and polydextrose may also be supplemented into the stabilized probiotic containing nutritional composition in a total amount of about 0.6 mg/100 Kcal.

The nutritional formulation in which stabilized probiotics of the present disclosure may be combined there with, may contain a source of long chain polyunsaturated fatty acids (LCPUFAs) which comprise docosahexanoic acid (DHA). Other suitable LCPUFAs include, but are not limited to, α-linoleic acid, Y-linoleic acid, linoleic acid, linolenic acid, eicosapentanoic acid (EPA) and arachidonic acid (ARA).

In one embodiment, the final composition may be supplemented with both DHA and ARA. In this embodiment, the weight ratio of ARA:DHA may be from about 1:3 to about 9:1. In one embodiment this ratio is from about 1:2 to about 4:1.

The amount of long chain polyunsaturated fatty acids in the nutritional composition may vary from about 5 mg/100 kcal to about 100 mg/100 kcal, more preferably from about 10 mg/100 kcal to about 50 mg/100 kcal.

A nutritional composition may be supplemented with oils containing DHA and ARA using standard techniques known in the art. For example, DHA and ARA may be added to the formula by replacing an equivalent amount of an oil, such as high oleic sunflower oil, normally present in the formula. As another example, the oils containing DHA and ARA may be added by replacing an equivalent amount of the rest of the overall fat blend normally present in the formula without DHA and ARA.

If utilized, the source of DHA and ARA may be any source known in the art such as marine oil, fish oil, single cell oil, egg yolk lipid, and brain lipid. In some compositions, the DHA and ARA are sourced from the single cell Martek oil, DHASCO®, or variations thereof. The DHA and ARA can be in natural form, provided that the remainder of the LCPUFA source does not result in any substantial deleterious effect on the infant. Alternatively, the DHA and ARA can be used in refined form.

In an embodiment of the nutritional composition combined with stabilized probiotics, sources of DHA and ARA are single cell oils as taught in U.S. Patent Nos. 5,374,567; 5,550,156; and 5,397,591.

In certain embodiments, the nutritional composition may be a milk-based nutritional composition which provides physiochemical and physiological benefits. As is known in the art, bovine milk protein comprises two major components: acid soluble whey protein and acid insoluble casein, with the latter representing about 80% of the total protein content of bovine milk. Upon entering the acidic environment of the stomach, casein precipitates and complexes with minerals forming semi-solid curds of varying size and firmness. Softer, smaller curds are easier for the body to digest than larger, harder curds. Curd formation may be an important consideration in the development of nutritional compositions, including, but not limited to infant formulas, medical foods, and premature infant formulas. As such, stabilized probiotics may be combined with compositions that include softer and smaller curds than standard infant formulas.

In other embodiments the stabilized probiotics of the present disclosure may be combined with nutritional compositions including bovine lactoferrin which retains its stability and activity in the human gut against certain undesirable bacterial pathogens. The nutritional composition of the disclosure may also contain TGF-β. In nutritional compositions, the level of TGF-β may be from about 0.0150 (pg/µg) ppm to about 0.1000 (pg/µg) ppm. In other nutritional compositions, the level of TGF-β in final composition including a stabilized probiotic is from about 0.0225 (pg/µg) ppm to about 0.0750 (pg/µg) ppm.

In some compositions, the level of TGF-β is from about 2500 pg/mL to about 10,000 pg/mL, more preferably from about 3000 pg/mL to about 8000 pg/mL.

In one embodiment, the ratio of TGF-β1: TGF-B2 is in the range of about 1:1 to about 1:20, or, more particularly, in the range of about 1:5 to about 1:15.

In some embodiments, the bioactivity of TGF-β in a nutritional composition is enhanced by the addition of a bioactive whey fraction. Any bioactive whey fraction known in the art may be used in this embodiment provided it achieves the intended result. In an embodiment, this bioactive whey fraction may be a whey protein concentrate. In a particular embodiment, the whey protein concentrate may be Salibra® 800, available from Glanbia Nutritionals. In a particular embodiment, the Salibra® 800 whey protein concentrate is 2.5% acidified. In another embodiment, the Salibra® 800 whey protein concentrate is 5% acidified. In yet another embodiment, the Salibra® 800 whey protein concentrate is 2% acidified. In a further embodiment, the Salibra® 800 whey protein concentrate is 3% acidified.

In another embodiment, the whey protein concentrate may be Nutri Whey 800, available from DMV International. In yet another embodiment, the whey protein concentrate may be Salibra-850, available from Glanbia Nutritionals. In still another embodiment, the whey protein concentrate may be Prolacta Lactalis WPI90, available from Lactilus Industries U.S.A., Inc. In a further embodiment, the whey protein concentrate may be supplied by MG Nutritionals.

Accordingly, by the practice of the present disclosure, stabilized probiotics having heretofore unrecognized stability are prepared. The stabilized bacterial mixture exhibits exceptionally high stability through the use of hydrolyzed mammalian protein, especially hydrolyzed mammalian protein with over 70% of the peptides having a molecular weight of less than 2,000 Daltons. The stabilized probiotics are uniquely effective for nutritional applications with intermediate moisture levels (such as water activity as high as 0.4) where increased shelf life and stability in hot and humid environments are desired. The stabilized probiotics may be packed separately or be combined with any of the embodiments of nutritional compositions described herein.

Although preferred embodiments of the disclosure have been described using specific terms, devices, and methods, such description is for illustrative purposes only. The words used are words of description rather than of limitation. It is to be understood that changes and variations may be made by those of ordinary skill in the art without departing from the scope of the present disclosure, which is set forth in the following claims. In addition, it should be understood that aspects of the various embodiments may be interchanged both in whole or in part. For example, while methods for the production of a commercially sterile liquid nutritional supplement made according to those methods have been exemplified, other uses are contemplated.

## Claims

1. An ingestible composition comprising:
a stabilized probiotic contained in a mixture of hydrolyzed mammalian protein, wherein the hydrolyzed protein comprises from about 10 percent to about 20 percent of the mixture components on a dry basis and at least 20% of the total hydrolyzed mammalian protein is comprised of protein having a molecular weight of less than 2000 Daltons;
one or more carbohydrates; and
a compound binder comprising sodium alginate.

2. The composition of claim 1 wherein the probiotic comprises viable microbial cells.

3. The composition of claim 2 wherein the viable microbial cells comprise *Lactobacillus rhamnosus.*

4. The composition of claim 1 wherein the hydrolyzed mammalian protein comprises hydrolyzed casein.

5. The composition of claim 1 wherein at least 70% of the total hydrolyzed mammalian protein is comprised of protein having a molecular weight of less than 2000 Daltons.

6. The composition of claim 1 wherein at least 90% of the total hydrolyzed mammalian protein is comprised of protein having a molecular weight of less than 2000 Daltons.

7. The ingestible composition of claim 1 wherein the probiotic comprises a non-viable probiotic.

8. The ingestible composition of claim 7, wherein the non-viable probiotic has been heat-killed.

9. The ingestible composition of claim 1, wherein the composition is an infant formula.

## Patentansprüche

1. Einnehmbare Zusammensetzung, umfassend:
ein stabilisiertes Probiotikum, das in einem Gemisch von hydrolysiertem Säugetierprotein enthalten ist, wobei das hydrolysierte Protein von etwa 10 Prozent bis etwa 20 Prozent der Komponenten des Gemischs auf Trockenbasis umfasst und wenigstens 20 % des gesamten hydrolysierten Säugetierproteins aus Protein mit einem Molekulargewicht von weniger als 2000 Dalton bestehen;
ein oder mehrere Kohlenhydrate; und
ein zusammengesetztes Bindemittel, das Natriumalginat umfasst.

2. Zusammensetzung gemäß Anspruch 1, wobei das Probiotikum lebensfähige mikrobielle Zellen umfasst.

3. Zusammensetzung gemäß Anspruch 2, wobei die lebensfähigen mikrobiellen Zellen *Lactobacillus rhamnosus* umfassen.

4. Zusammensetzung gemäß Anspruch 1, wobei das hydrolysierte Säugetierprotein hydrolysiertes Kasein umfasst.

5. Zusammensetzung gemäß Anspruch 1, wobei wenigstens 70 % des gesamten hydrolysierten Säugetierproteins aus Protein mit einem Molekulargewicht von weniger als 2000 Dalton bestehen.

6. Zusammensetzung gemäß Anspruch 1, wobei wenigstens 90 % des gesamten hydrolysierten Säugetierproteins aus Protein mit einem Molekulargewicht von weniger als 2000 Dalton bestehen.

7. Einnehmbare Zusammensetzung gemäß Anspruch 1, wobei das Probiotikum ein nicht lebensfähiges Probiotikum umfasst.

8. Einnehmbare Zusammensetzung gemäß Anspruch 7, wobei das nicht lebensfähige Probiotikum durch Hitze abgetötet worden ist.

9. Einnehmbare Zusammensetzung gemäß Anspruch 1, wobei die Zusammensetzung eine Säuglingsnahrung ist.

## Revendications

1. Composition pour ingestion comprenant :
un probiotique stabilisé contenu dans un mélange de protéine mammalienne hydrolysée, où la protéine hydrolysée comprend entre environ 10 pour cent et environ 20 pour cent des composants du mélange sur la base de la matière sèche et au moins 20 % du total de la protéine mammalienne hydrolysée sont constitués d'une protéine de masse moléculaire inférieure à 2000 daltons ;
un ou plusieurs hydrates de carbone ; et
un agent liant complexe comprenant de l'alginate de sodium.

2. Composition selon la revendication 1, où le probiotique comprend des cellules microbiennes viables.

3. Composition selon la revendication 2, où les cellules microbiennes viables comprennent *Lactobacillus rhamnosus.*

4. Composition selon la revendication 1, où la protéine mammalienne hydrolysée comprend de la caséine hydrolysée.

5. Composition selon la revendication 1, où au moins 70 % du total de la protéine mammalienne hydrolysée sont constitués d'une protéine de masse moléculaire inférieure à 2000 daltons.

6. Composition selon la revendication 1, où au moins 90 % du total de la protéine mammalienne hydrolysée sont constitués d'une protéine de masse moléculaire inférieure à 2000 daltons.

7. Composition pour ingestion selon la revendication 1, où le probiotique comprend un probiotique non viable.

8. Composition pour ingestion selon la revendication 7, où le probiotique non viable a été détruit à la chaleur.

9. Composition pour ingestion selon la revendication 1, où la composition est une formule pour nourrissons.
